# EUROPÄISCHE PATENTSCHRIFT

(11) **EP 0 787 141 B1**
(45) Veröffentlichungstag und Bekanntmachung des Hinweises auf die Patenterteilung: **30.08.2000**
(21) Anmeldenummer: 95935902.7
(22) Anmeldetag: 05.10.1995
(51) Int. Cl.: C07K 11/02, C07D 273/00, A61K 31/38

(54) **VERFAHREN ZUR SULFONYLIERUNG, SULFENYLIERUNG UND PHOSPHORYLIERUNG VON CYCLISCHEN DEPSIPEPTIDEN**
CYCLIC DEPSIPEPTIDE SULFONYLATION, SULFENYLATION AND PHOSPHORYLATION PROCESS
PROCEDE DE SULFONYLATION, DE SULFENYLATION ET DE PHOSPHORYLATION DE DEPSIPEPTIDES CYCLIQUES

(30) Priorität: 18.10.1994 DE 4437198
(43) Veröffentlichungstag der Anmeldung: 06.08.1997
(73) Patentinhaber: BAYER AG, 51368 Leverkusen (DE)
(72) Erfinder: SCHERKENBECK, Jürgen, D-42929 Wermelskirchen (DE); PLANT, Andrew, D-51373 Leverkusen (DE); JESCHKE, Peter, D-51383 Leverkusen (DE); HARDER, Achim, D-51109 Köln (DE); MENCKE, Norbert, D-51381 Leverkusen (DE)
(86) Internationale Anmeldenummer: EP9503926
(87) Internationale Veröffentlichungsnummer: WO9611945

(56) Entgegenhaltungen:
- EP-A- 0 382 173
- EP-A- 0 626 376
- PROCEEDINGS OF NOBEL SYMPOSIUM, Bd. 34, 1976, STOCKHOLM, Seiten 345-372, XP002005330 YU.A.OVCHINNIKOV: "Recent Findings in the Structural and Functional Aspects of the Peptide Ionophores"
- INT.J.PEPTIDE PROTEIN RES., Bd. 12, 1978, COPENHAGEN, Seiten 7-16, XP002005331 Y.SHIMOHIGASHI ET AL.: "Cyclic Peptides"

## Beschreibung

Die vorliegende Erfindung betrifft sulfonylierte, sulfenylierte, thiocyanierte und phosphorylierte cyclische Depsipeptide sowie Verfahren zu deren Herstellung und deren Verwendung als Endoparasitizide.

Cyclische Depsipeptide und ihre Wirkung als Endoparasitizide sind bereits bekannt aus EP 382 173, PCT WO 93/19 053. Die Wirkung dieser Verbindungen ist jedoch nicht in jedem Fall befriedigend.

Weiterhin beschreibt Proceedings of Nobel Symposium, Vol. 34, S. 345-72 (1976) Stockholm, die ionophoretischen Eigenschaften von Enniatinen, es wird jedoch nichts über deren Tauglichkeit als Endoparasitizide gesagt.

In Int. J. Peptide Protein Res., Vol. 12, S. 7-16 (1978) werden zwar cyclische Peptide vorgestellt, es wird jedoch nichts über cyclische Depsipeptide gesagt.

Cyclische Depsipeptide, die als Ausgangsstoffe für das erfindungsgemäße Verfahren eingesetzt werden, sind üblicherweise fermentativ hergestellte Naturstoffe. Von ihrer Struktur her sind sie vergleichbar mit Proteinen. Man würde daher erwarten, daß sie gegenüber aggressiven Chemikalien empfindlich reagieren und ganz oder zumindest teilweise zerstört werden. Überraschenderweise wurde gefunden, daß das erfindungsgemäße Verfahren durchgeführt werden kann, ohne den Grundkörper der Depsipeptide zu zerstören. Man gelangt so zu Verbindungen, die am Phenylring sulfonyliert, sulfenyliert, thiocyaniert oder phosphoryliert sind und die sich durch hervorragende endoparasitizide Wirkung auszeichnen.

Gegenstand der vorliegenden Erfindung sind Verbindungen der allgemeinen Formel (I) 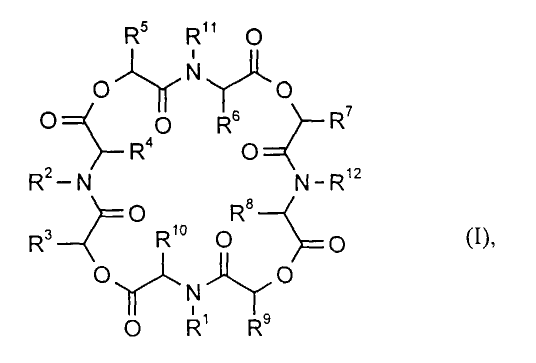 in welcher mindestens einer der Reste
- R³, R⁴, R⁵, R⁶, R⁷, R⁸, R⁹, R¹⁰: für Benzyl steht, das durch einen Sulfonyl-, Sulfenyl-, Thiocyan- oder Phosphorylrest substituiert ist
und in welcher ansonsten die Reste
- R¹, R², R¹¹ und R¹²: für Wasserstoff, gegebenenfalls substituierte Reste C₁₋₈-Alkyl, C₃₋₆-Cycloalkyl, Benzyl, Phenylethyl, Phenyl stehen,
- R³, R⁵, R⁷, R⁹: für Wasserstoff, geradkettiges oder verzweigtes C₁₋₈-Alkyl, das gegebenenfalls durch Hydroxy, C₁₋₄-Alkoxy, Phenoxy, Carboxy, 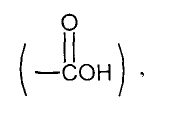 Carboxamid, 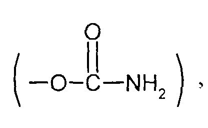 Imidazolyl, Indolyl, Guanidino, -SH oder C₁₋₄-Alkylthio substituiert sein kann, ferner für gegebenenfalls substituiertes Phenyl, Benzyl oder Phenylethyl stehen,
- R⁴, R⁶, R⁸, R¹⁰: für Wasserstoff, geradkettiges oder verzweigtes C₁₋₈-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für gegebenenfalls substituiertes Phenyl, Benzyl oder Phenylethyl stehen.

Als Sulfonyl-, Sulfenyl-, Thiocyan- oder Phosphorylreste seien genannt: -SO₂-A; SO-A, -S-A, 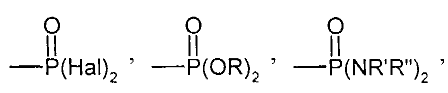 SCN
wobei
- Hal: für Halogen steht,
- A für Halogen, Hydroxy, -OR, -NH₂, -NHR', -NR'R" steht,
- R: für gegebenenfalls substituiertes C₁₋₈-Alkyl, C₂₋₆-Alkenyl, Phenyl, Benzyl oder Phenylethyl steht,
- R': für gegebenenfalls substituiertes C₁₋₈-Alkyl, Phenyl, Benzyl oder Phenylethyl steht,
- R": für gegebenenfalls substituiertes C₁₋₈-Alkyl, Phenyl, Benzyl oder Phenylethyl steht,
und die Reste R' und R" gemeinsam mit dem angrenzenden Stickstoffatom für einen Morpholinrest, Piperazinrest, oder N-Methylpiperazinrest stehen, und in welcher ansonsten die Reste R¹, R², R⁴, R⁵, R⁶, R⁸, R⁹, R¹⁰, R¹¹, R¹² die in Anspruch 1 angegebene Bedeutung haben;
die gegebenenfalls substituierten Reste können 1 bis 2 gleiche oder verschiedene Substituenten tragen. Als Substituenten seien aufgeführt:

Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind und als Halogenatome Fluor, Chlor oder Brom stehen; Hydroxy; Halogen, Cyano; Nitro; Amino; Monoalkyl- und Dialkylamino mit 1 bis 4 Kohlenstoffatomen je Alkylgruppe.

Gegebenenfalls substituiertes Alkyl allein oder als Bestandteil eines Restes in den allgemeinen Formeln bedeutet geradkettiges oder verzweigtes Alkyl mit vorzugsweise 1 bis 8, insbesondere 1 bis 5 Kohlenstoffatomen. Beispielhaft und vorzugsweise seien gegebenenfalls substituiertes Methyl, Ethyl, n- und i-Propyl, n-, i- und t-Butyl, genannt.

Gegebenenfalls substituiertes Alkenyl allein oder als Bestandteil eines Restes in den allgemeinen Formeln bedeutet geradkettiges oder verzweigtes Alkenyl mit vorzugsweise 2 bis 6, insbesondere 2 bis 4 Kohlenstoffatomen. Beispielhaft und vorzugsweise seien gegebenenfalls substituiertes Ethenyl, Propenyl-(1), Propenyl-(2) und Butenyl-(3) genannt.

Gegebenenfalls substituiertes Cycloalkyl in den allgemeinen Formeln bedeutet mono-, bi- und tricyclisches Cycloalkyl mit vorzugsweise 3 bis 6, insbesondere 3, 5 oder 6 Kohlenstoffatomen. Beispielsweise und vorzugsweise seien gegebenenfalls substituiertes Cyclopropyl, Cyclobutyl, Cyclopentyl, Cyclohexyl genannt.

Gegebenenfalls substituiertes Alkoxy in den allgemeinen Formeln bedeutet geradkettiges oder verzweigtes Alkoxy mit vorzugsweise 1 bis 6, insbesondere 1 bis 4 Kohlenstoffatomen. Beispielhaft und vorzugsweise seien gegebenenfalls substituiertes Methoxy, Ethoxy, n- und i-Propoxy und n-, o- und t-Butoxy genannt.

Gegebenenfalls substituiertes Alkylthio in den allgemeinen Formeln bedeutet geradkettiges oder verzweigtes Alkylthio mit vorzugsweise 1 bis 6, insbesondere 1 bis 4 Kohlenstoffatomen. Beispielhaft und vorzugsweise seien gegebenenfalls substituiertes Methylthio, Ethylthio, n- und i-Propylthio, n-, o- und t-Butylthio genannt.

Halogenalkyl in den allgemeinen Formeln enthält 1 bis 4, insbesondere 1 oder 2 Kohlenstoffatome und vorzugsweise 1 bis 9, insbesondere 1 bis 5 gleiche oder verschiedene Halogenatome, wobei als Halogenatome vorzugsweise Fluor, Chlor und Brom, insbesondere Fluor und Chlor stehen. Beispielhaft seien Trifluormethyl, Chlor-di-fluormethyl, 2,2,2-Trifluorethyl und Pentafluormethyl, Perfluor-t-butyl genannt.

Gegebenenfalls substituiertes Aryl in den allgemeinen Formeln bedeutet vorzugsweise gegebenenfalls substituiertes Phenyl oder Naphthyl, insbesondere Phenyl.

Gegebenenfalls substituiertes Aralkyl in den allgemeinen Formeln bedeutet gegebenenfalls im Arylteil und/oder Alkylteil substituiertes Aralkyl mit vorzugsweise 6 oder 10, insbesondere 6 Kohlenstoffatomen im Arylteil (vorzugsweise Phenyl oder Naphthyl, insbesondere Phenyl) und vorzugsweise 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl genannt.

Gegebenenfalls substituiertes Hetaryl allein oder als Bestandteil eines Restes bedeutet in den allgemeinen Formeln 5- bis 7-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Beispielhaft und vorzugsweise seien gegebenenfalls substituiertes Furyl, Thiophenyl, Pyrazolyl, Imidazolyl, 1,2,3- und 1,2,4-Triazolyl, Oxazolyl, Isoxazolyl, Thiazolyl, Isothiazolyl, 1,2,3-, 1,3,4-, 1,2,4- und 1,2,5-Oxadiazolyl, Azepinyl, Pyrrolyl, Isopyrrolyl, Pyridyl, Piperazinyl, Pyridazinyl, Pyrimidinyl, Pyrazinyl, 1,3,5-, 1,2,4- und 1,2,3-Triazinyl, 1,2,4-, 1,2,3-, 1,3,6- und 1,2,6-Oxazinyl, Oxepinyl, Thiepinyl und 1,2,4-Diazepinyl genannt.

Die gegebenenfalls substituierten Reste der allgemeinen Formeln können 1 bis 2 gleiche oder verschiedene Substituenten tragen. Als Substituenten seien aufgeführt:

Alkyl mit 1 bis 4, insbesondere 1 oder 2 Kohlenstoffatomen, wie Methyl, Ethyl, n- und i-Propyl und n-, i- und t-Butyl; Alkoxy mit 1 bis 4, insbesondere 1 oder 2 Kohlenstoffatomen, wie Methoxy, Ethoxy, n- und i-Propyloxy und n-, i- und t-Butyloxy; Alkylthio mit 1 bis 4, insbesondere 1 oder 2 Kohlenstoffatomen, wie Methylthio, Ethyltho, n- und i-Propylthio und n-, i- und t-Butylthio; Halogenalkyl mit 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Difluormethyl, Trifluormethyl; Hydroxy; Halogen, vorzugsweise Fluor, Chlor, Brom, insbesondere Fluor, Chlor, Cyano; Nitro; Amino; Monoalkyl- und Dialkylamino mit 1 bis 4, insbesondere 1 oder 2 Kohlenstoffatomen je Alkylgruppe, wie Methylamino, Methylethylamino, n- und i-Propylamino und Methyl-n-Butylamino; Acylreste wie Carboxyl; Carboxy mit vorzugsweise 2 bis 4, insbesondere 2 oder 3 Kohlenstoffatomen, wie Carbomethoxy und Carboethoxy; Alkylsulfinyl mit 1 bis 4, insbesondere 1 bis 2 Kohlenstoffatomen, Halogenalkylsulfinyl mit 1 bis 4, insbesondere 1 bis 2 Kohlenstoffatomen und 1 bis 5 Halogenatomen wie Trifluonnethylsulfinyl mit 1 bis 4, insbesondere 1 bis 2 Kohlenstoffatomen mit 1 bis 5 Halogenatomen wie Trifluormethylsulfinyl; Sulfonyl (-SO₃H); Alkylsulfonyl mit vorzugsweise 1 bis 4, insbesondere 1 oder 2 Kohlenstoffatomen, wie Methylsulfonyl und Ethylsulfonyl; Halogenalkylsulfonyl mit 1 bis 4, insbesondere 1 bis 2 Kohlenstoffatomen und 1 bis 5 Halogenatomen wie Triflormethylsulfonyl, Perfluor-t,n,s-butylsulfonyk; Arylsulfonyl mit vorzugsweise 6 oder 10 Arylkohlenstoffatomen, wie Phenylsulfonyl; Acyl, Aryl, Aryloxy, Heteroaryl, Heteroaryloxy, die ihrerseits einen der oben genannten Substituenten tragen können sowie der Formiminorest

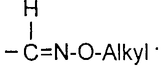

Bevorzugt werden Verbindungen der Formel (I) eingesetzt, in welcher
- R¹, R², R¹¹ und R¹²: unabhängig voneinander für Methyl, Ethyl, Propyl, Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können;
- R³ bis R¹⁰: die oben angegebene Bedeutung haben.

Besonders bevorzugt sind Verbindungen der Formel (I), in welcher
- R¹, R², R¹¹ und R¹²: unabhängig voneinander für Methyl, Ethyl, Propyl, Isopropyl oder n-, s-, t-Butyl stehen,
wobei mindestens einer der Reste R³ oder R¹⁰ für Benzyl steht, dessen Phenylrest durch einen der oben angegebenen Sulfonyl-, Sulfenyl- oder Phosphorylreste substituiert ist.

Die neuen aromatischen sulfonylierten, sulfenylierten, thiocyanierten oder phosphorylierten cyclischen Depsipeptide der Formel (I) sowie deren Säureadditions-Salze und Metallsalz-Komplexe besitzen sehr gute anthelminthische Eigenschaften und können bevorzugt im Bereich der Veterinärmedizin eingesetzt werden. Überraschenderweise zeigen sie bei der Bekämpfung von Wurmerkrankungen eine bessere Wirksamkeit konstitutionell ähnliche, vorbekannte Verbindungen gleicher Wirkungsrichtung.

Ebenfalls Gegenstand dieser Erfindung ist ein Verfahren zur aromatischen Sulfonylierung, Sulfenylierung, Thiocyanierung und Phosphorylierung von cyclischen Depsipeptiden der allgemeinen Formel (I) 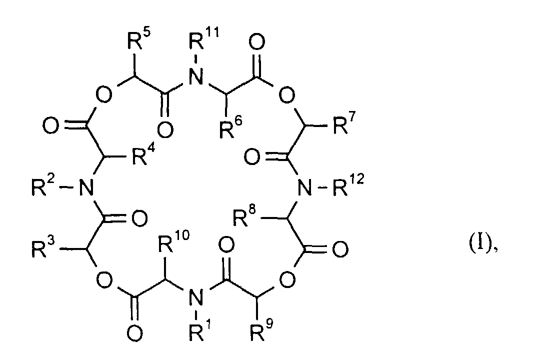 in welcher mindestens einer der Reste
- R³, R⁴, R⁵, R⁶, R⁷, R⁸, R⁹, R¹⁰: für Benzyl steht,
und in welcher ansonsten die Reste
- R¹, R², R¹¹ und R¹²: für Wasserstoff, gegebenenfalls substituierte Reste C₁₋₈-Alkyl, C₃₋₆-Cycloalkyl, Benzyl, Phenylethyl oder Phenyl stehen,
- R³, R⁵, R⁷, R⁹: für Wasserstoff, geradkettiges oder verzweigtes C₁₋₈-Alkyl, das gegebenenfalls durch Hydroxy, C₁₋₄-Alkoxy, Phenoxy, Carboxy, 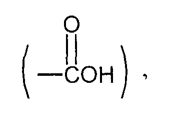 Carboxamid, 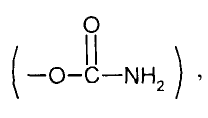 Imidazolyl, Indolyl, Guanidino, -SH oder C₁₋₄-Alkylthio substituiert sein kann, ferner für gegebenenfalls substituiertes Phenyl, Phenylethyl oder Benzyl stehen,
- R⁴, R⁶, R⁸, R¹⁰: für Wasserstoff, geradkettiges oder verzweigtes C₁₋₈-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für gegebenenfalls substituiertes Phenyl, Benzyl oder Phenylethyl stehen,
dadurch gekennzeichnet, daß man diese cyclischen Depsipeptide mit Sulfonylierungs-, Sulfenylierungs-, Thiocyanierungs- oder Phosphorylierungsreagenzien gegebenenfalls in Gegenwart von Katalysatoren, Hilfsstoffen und/oder Verdünnungsmitteln umsetzt.

Bevorzugt ist dabei der Einsatz von Depsipeptiden der Formel (I), in welcher R³ und R⁷ für Benzyl stehen und die übrigen Reste die oben genannte Bedeutung haben.

Zur Durchführung des erfindungsgemäßen Verfahrens werden als Sulfonylierungs- und Sulfenylierungsreagenzien Halogensulfonsäuren (HalSO₃H), insbesondere Chlorsulfonsäure, sowie deren Derivate, Dihalogensulfid, insbesondere Dichlorsulfid sowie seine Derivate, Halogensulfensäure, insbesondere Chlorsulfensäure, Sulfenylhalogenide, insbesondere Sulfenylchloride, Disulfide, Schwefelsäure (Oleum) gegebenenfalls in einem gegen die Reagenzien inerten Verdünnungsmittel sowie gegebenenfalls in Gegenwart von Lewis-Säuren eingesetzt.

Zur Durchführung des erfindungsgemäßen Verfahrens werden als Thiocyanierungsreagenzien Rhodanidsalze, insbesondere Kupfer(II)rhodanid und Ammoniumrhodanid oder Dirhodan ((SCN)₂), gegebenenfalls in einem gegen die Reagenzien inerten Verdünnungsmittel sowie gegebenenfalls in Gegenwart von Lewis-Säuren eingesetzt.

Zur Durchführung des erfindungsgemäßen Verfahrens werden als Phosphorylierungsreagenzien Phosphorhalogenide, insbesondere Phosphortrichlorid, Phosphorpentachlorid, gegebenenfalls in einem gegen die Reagenzien inerten Verdünnungsmittel sowie gegebenenfalls in Gegenwart von Lewis-Säuren eingesetzt.

Als Lewis-Säuren werden genannt: AlCl₃, TiCl₄, BF₃ OEt₂, SbCl₅, SnCl₄, CuCl₂, FeCl₃, SnCl₂, AgBF₄, AgSbF₆, AgClO₄, LiClO₄, Br₂.

Die Umsetzung erfolgt bei Temperaturen von 0 bis 150°C, bevorzugt bei 0 bis 80°C, besonders bevorzugt bei 0 bis 60°C.

Als Verdünnungsmittel kommen alle gegenüber den Reagenzien inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylether-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüberhinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Die Depsipeptide werden mit einem Überschuß Reagenz (5 bis 10 Äquivalente) und einem Überschuß Lewis-Säure (15 bis 20 Äquivalente) umgesetzt.

Nach erfolgter Umsetzung wird das Verdünnungsmittel abdestilliert und die sulfonylierten, sulfenylierten, thiocyanierten oder phosphorylierten Verbindungen der Formel (I) in üblicher Weise, z.B. chromatographisch, gereinigt.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todefälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparsiten zählen Cesthoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß.Forrnulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder linerares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffe, die gegen pathogene Endoparasiten wirken, vorliegen. Solche Wirkstoffe sind z.B. L-2,3,5,6-Tetrahydro-6-phenyl-imidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1-10 Gewichtsprozent.

Zubereitungen die vor Anwendung verdünnt werden, enthalten den Wirkstoff in Konzentrationen von 0,5-90 Gew.-%, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

### Haemonchus contortus / Schaf

Experimentell mit Heamonchus contortus infizierte Schafe wurden nach Ablauf der Präpatenzzeit des Parasiten behandelt. Die Wirkstoffe wurden als reiner Wirkstoff in Gelatinekapseln oral appliziert (p.o) oder als Lösung intravenös injiziert (i.v.).

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Beispiel Nr. | Dosis effectiva in mg/kg | |
|---|---|---|
| | p.o | i.v. |
| 1 | 0,5 | 0,5 |

Die Herstellung der erfindungsgemäßen Wirkstoffe geht aus den nachfolgenden Beispielen hervor.

### Herstellungsbeispiele

### 1. Allgemeine Verfahrensschrift für die Chlorsulfonierung

Eine Lösung eines Depsipeptids (0,523 mmol) in Dichlormethan wird bei 0°C mit Chlorsulfonsäure (37,3 mmol) versetzt, 2 Stunden bei 0°C und 2 Stunden bei Raumtemperatur gerührt. Das Reaktionsgemisch wird bei 0°C in Aceton (50 ml) eingetropft. Anschließend wird bei 0°C mit Morpholin (79,4 mmol) versetzt und 12 Stunden bei 60°C nachgerührt. Nach dieser Zeit wird eingeengt, in Wasser aufgenommen und mit Dichlormethan extrahiert (3 x). Die vereinigten organischen Extrakte werden über Na₂SO₄ getrocknet und eingeengt. Der Rückstand wird säulenchromatographisch mit dem Laufmittel tert.-Butylmethylether-Cyclohexan-Methanol = 10:1:0,4 gereinigt. Es werden Depsipeptide der Formel (I) erhalten, in welcher die Substituenten die folgende Bedeutung haben.

### 2. Allgemeine Vorschrift für die Sulfonierung

Das entsprechende Depsipeptid (1,05 mmol) wird in 0°C kaltes Oleum (20 %, 25 ml) eingetragen und 1 bis 2 Stunden bei dieser Temperatur gerührt. Anschließend wird auf 250 ml Eis gegossen und mit Ba(OH)₂ neutralisiert. Der Rückstand wird abfiltriert und dreimal mit Wasser gewaschen. Das Filtrat wird auf 5 bis 10 ml eingeengt und über einen stark sauren Ionenaustauscher (50 g) mit Wasser chromatographiert. Nach Einengen des Lösungsmittels werden die Depsipeptide der Formel (I) erhalten.

### 3. Allgemeine Vorschrift für die Phosphorylierung

Zu einer auf -10°C gekühlten Suspension von PCl₃ (3 Äquivalente), AlCl₃ (3 Äquivalente) und dem Depsipeptid in Tetrachlormethan wird tropfenweise Sulfonylchlorid (3 Äquivalente) zugegeben. Es wird 1 Minute bei 70°C nachgerührt und überschüssiges SO₂Cl₂ im Vakuum abdestilliert. Der Rückstand wird in Tetrachlormethan aufgenommen und unter Kühlung langsam mit einem Überschuß eines Alkohols oder Amins versetzt. Das Reaktionsgemisch wird mehrfach mit wenig Wasser gewaschen, über Na₂SO₄ getrocknet und eingeengt. Säulenchromatographie des Rückstandes an Kieselgel liefert die Depsipeptide der Formel (I).

### 4. Allgemeine Vorschrift für die Sulfenylierung

SbCl₅ (0,15 mmol) und AgSbF₆ (0,15 mmol) werden in 2 ml 1,2-Dichlorethan bei Raumtemperatur vorgelegt, dann wird eine Löung von Dimethyldisulfid (0,5 mmol) in Dichlormethan (2 ml) zugetropft und zuletzt eine Lösung des Depsipeptids (1,0 mmol) in 1,2-Dichlorethan zugetropft. Das Reaktionsgemisch wird für 3 bis 6 Stunden zum Rückfluß erhitzt und nach Abkühlen mit ges. wäßriger NaHCO₃-Lösung gequenscht. Es wird dreimal mit Wasser gewaschen, über Na₂SO₄ getrocknet und eingeengt. Säulenchromatographie des Rückstandes an Kieselgel liefert die Depsipeptide der Formel (I).

### 5. Allgemeine Vorschrift für die Thiocyanierung

Eine Lösung des Depsipeptides (1,0 mmol) in Eisessig (5 ml) wird mit Ca(SCN)₂ (2 bis 5 mmol) umgesetzt und bei 60°C gerührt. Nach Abkühlen und Filtrieren wird mit Wasser verdünnt, mit NaHCO₃-Lösung neutralisiert und mit Ethylacetat extrahiert. Säulenchromatographie des Rückstandes an Kieselgel liefert die Depsipeptide der Formel (I).

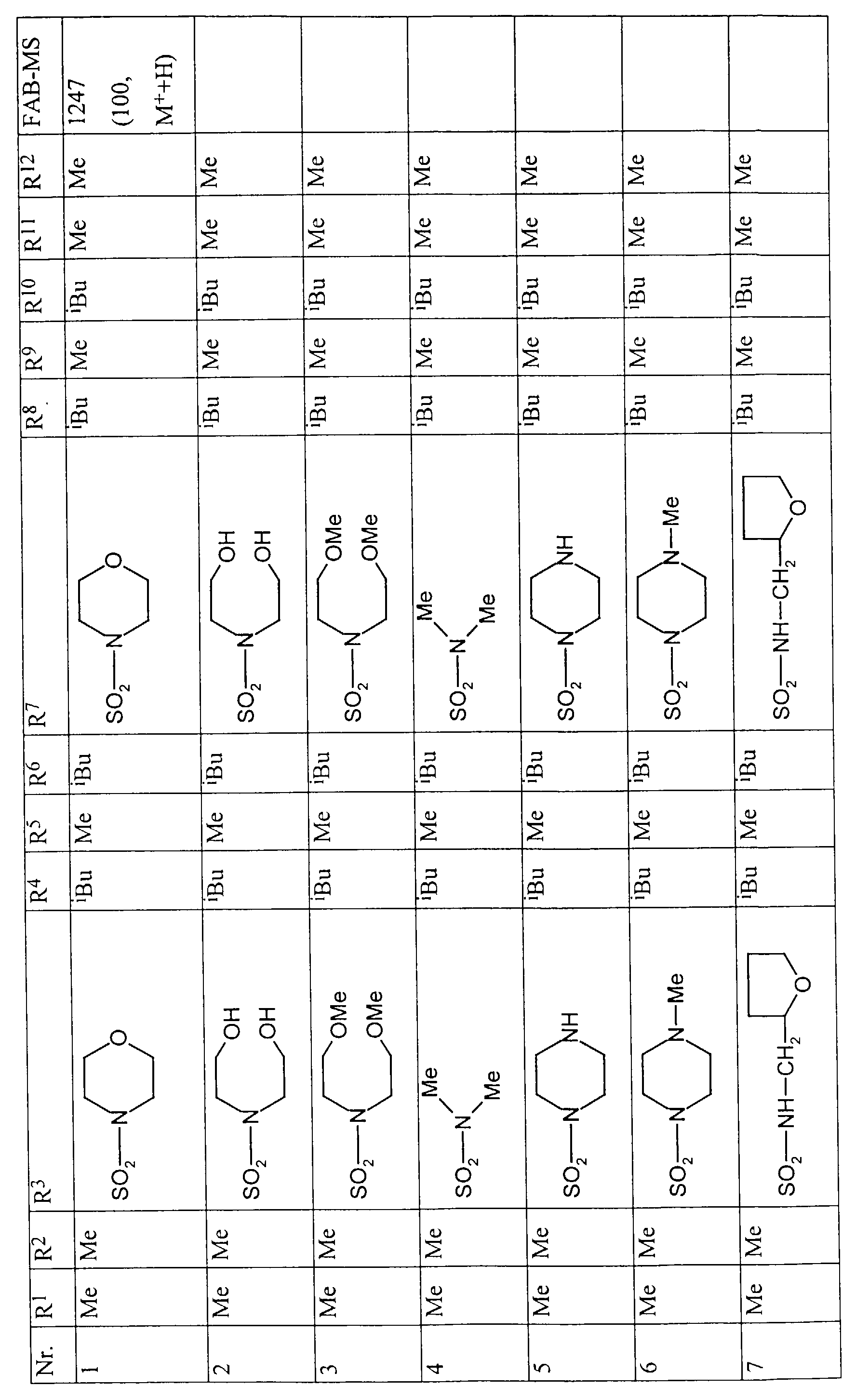

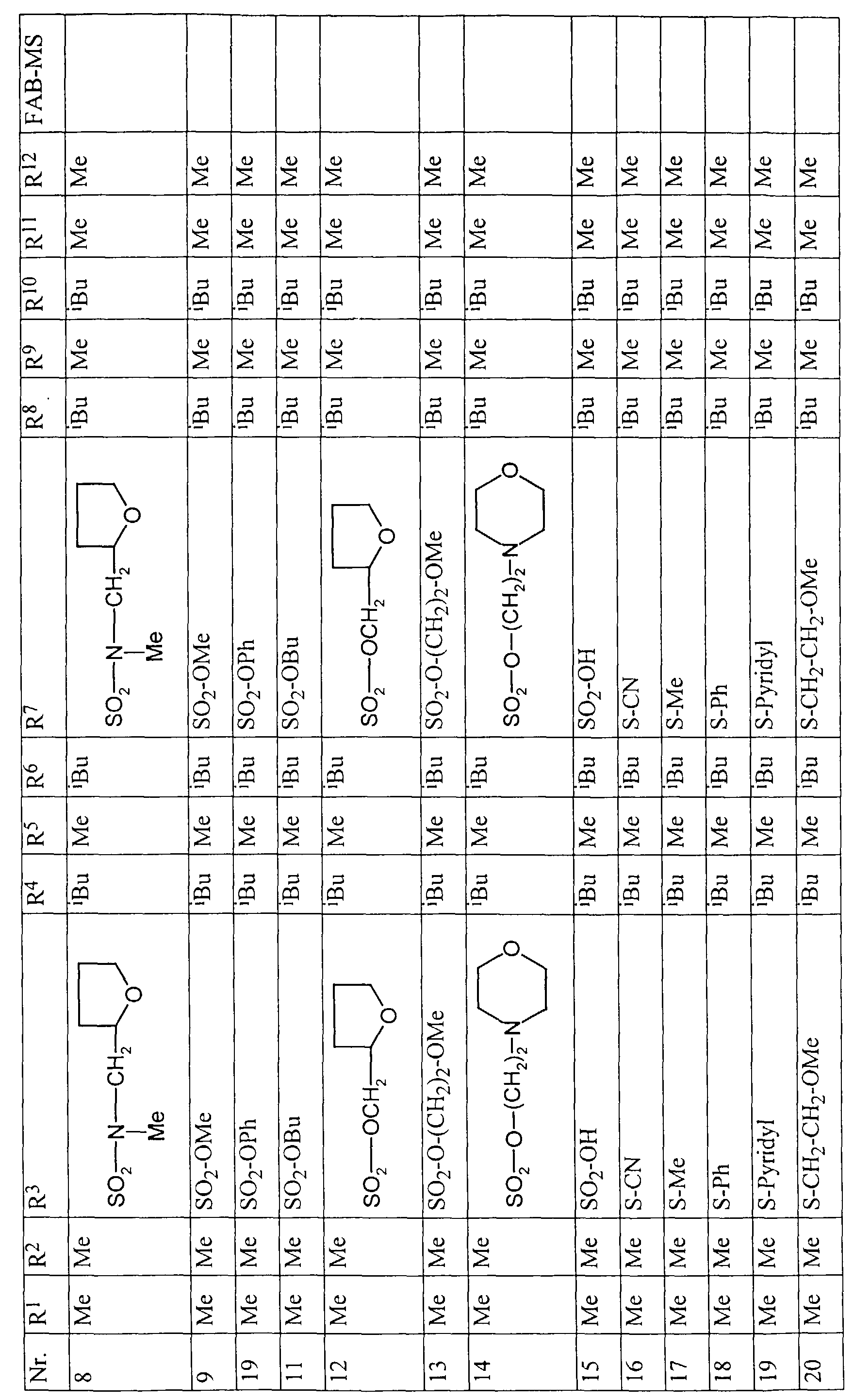

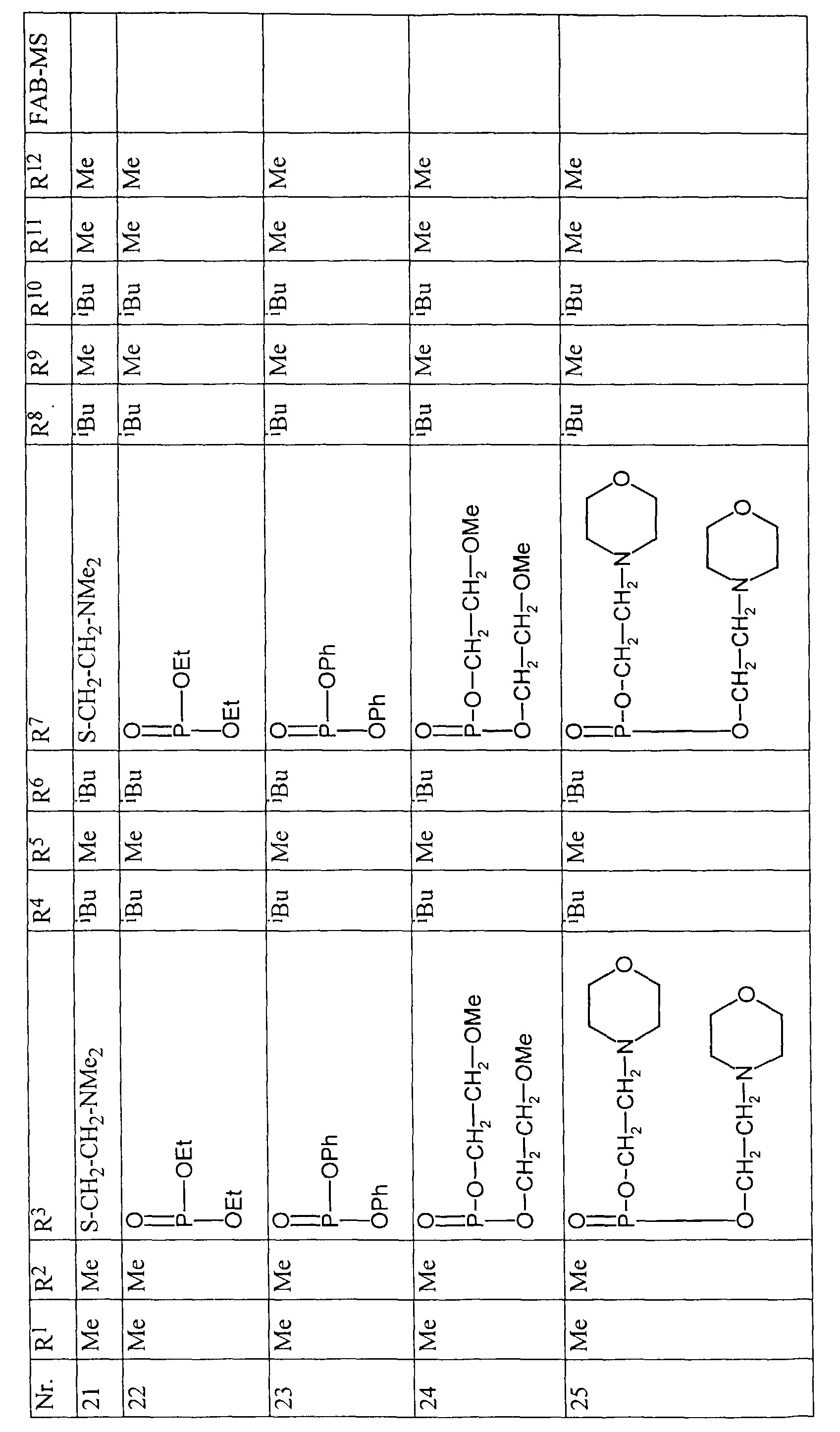

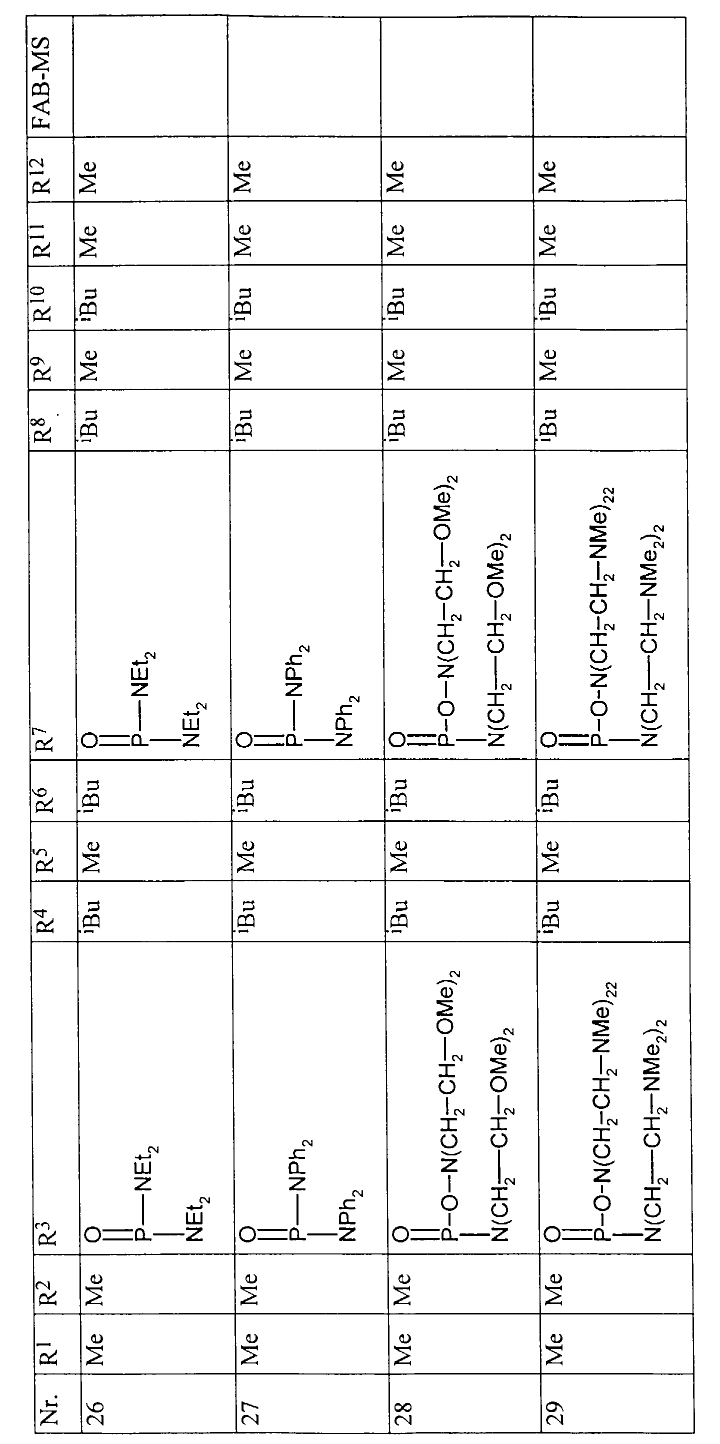

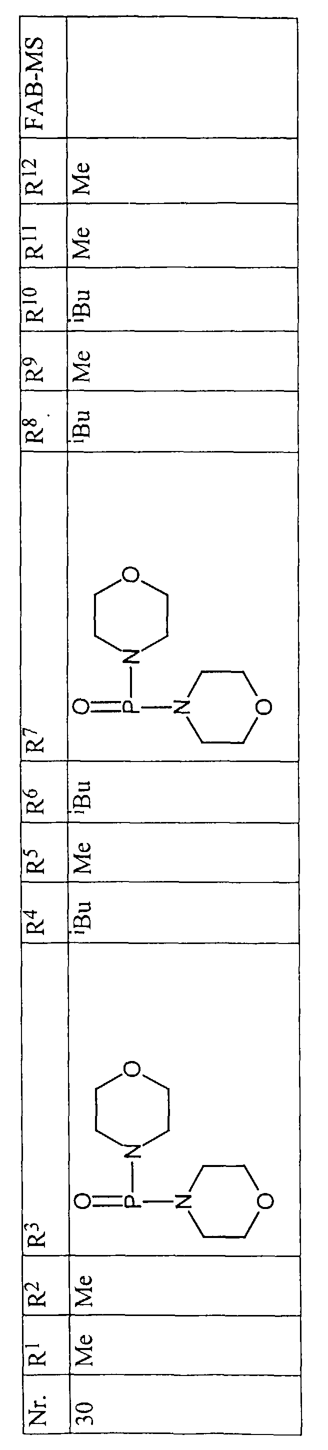

## Patentansprüche

1. Verbindungen der allgemeinen Formel (I) 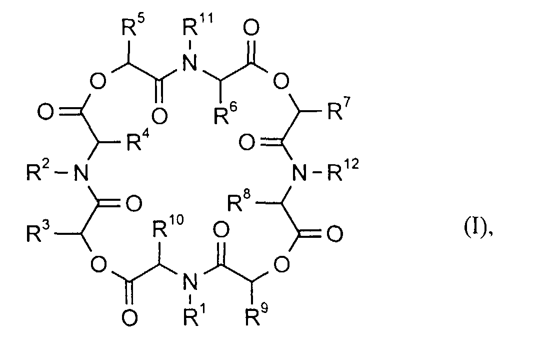 in welcher mindestens einer der Reste
R³, R⁴, R⁵, R⁶, R⁷, R⁸, R⁹, R¹⁰ für Benzyl steht, das durch einen Sulfonyl-, Sulfenyl-, Thiocyan- oder Phosphorylrest substituiert ist
und in welcher ansonsten die Reste
R¹, R², R¹¹ und R¹² für Wasserstoff, gegebenenfalls substituierte Reste C₁₋₈-Alkyl, C₁₋₆-Cycloalkyl, Benzyl, Phenylethyl, Phenyl stehen,
R³, R⁵, R⁷, R⁹ für Wasserstoff, geradkettiges oder verzweigtes C₁₋₈-Alkyl, das gegebenenfalls durch Hydroxy, C₁₋₄-Alkoxy, Phenoxy, Carboxy, 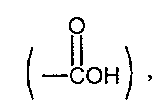 Carboxamid, 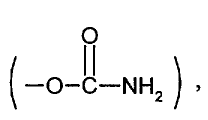 Imidazolyl, Indolyl, Guanidino, -SH
oder C₁₋₄-Alkylthio substituiert sein kann, ferner für gegebenenfalls substituiertes Phenyl, Benzyl oder Phenylethyl stehen,
R⁴, R⁶, R⁸, R¹⁰ für Wasserstoff, geradkettiges oder verzweigtes C₁₋₈-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für gegebenenfalls substituiertes Phenyl, Benzyl oder Phenylethyl stehen.

2. Verbindungen der Formel (I) gemäß Anspruch 1, in welcher die Reste R³ und R⁷ für Benzyl stehen, das durch einen Sulfonyl-, Sulfenyl-, Thiocyan- oder Phosphorylrest der Formeln
-SO₂-A; SO-A, -S-A, 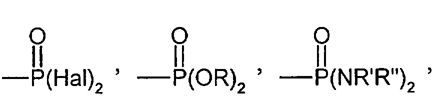 SCN substituiert ist,
wobei
Hal für Halogen steht,
A für Halogen, Hydroxy, -OR, -NH₂, -NHR', -NR'R" steht,
R für gegebenenfalls substituiertes C₁₋₈-Alkyl, C₂₋₆-Alkenyl, Phenyl, Benzyl oder Phenylethyl steht,
R' für gegebenenfalls substituiertes C₁₋₈-Alkyl, Phenyl, Benzyl oder Phenylethyl steht,
R" für gegebenenfalls substituiertes C₁₋₈-Alkyl, Phenyl, Benzyl oder Phenylethyl steht,
und die Reste R' und R" gemeinsam mit dem angrenzenden Stickstoffatom für einen Morpholinrest, Piperazinrest, oder N-Methylpiperazinrest stehen, und in welcher ansonsten die Reste R¹, R², R⁴, R⁵, R⁶, R⁸, R⁹, R¹⁰, R¹¹, R¹² die in Anspruch 1 angegebene Bedeutung haben;
die gegebenenfalls substituierten Reste können 1 bis 2 gleiche oder verschiedene Substituenten tragen. Als Substituenten seien aufgeführt:
Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind und als Halogenatome Fluor, Chlor oder Brom stehen; Hydroxy; Halogen, Cyano; Nitro; Amino; Monoalkyl- und Dialkylamino mit 1 bis 4 Kohlenstoffatomen je Alkylgruppe.

3. Verfahren zur aromatischen Sulfonylierung, Sulfenylierung, Thiocyanierung und Phosphorylierung von cyclischen Depsipeptiden der allgemeinen Formel (I) 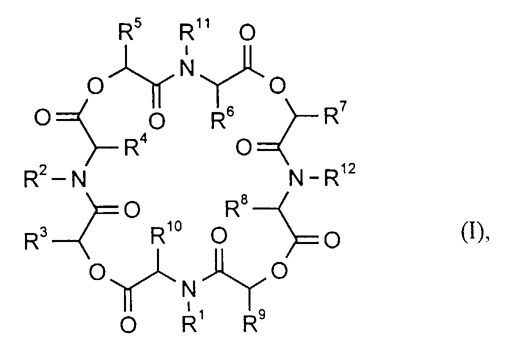 in welcher mindestens einer der Reste
R³, R⁴, R⁵, R⁶, R⁷, R⁸, R⁹, R¹⁰ für Benzyl steht,
und in welcher ansonsten die Reste
R¹, R², R¹¹ und R¹² für Wasserstoff, gegebenenfalls substituierte Reste C₁₋₈-Alkyl, C₃₋₆-Cycloalkyl, Benzyl, Phenylethyl oder Phenyl stehen,
R³, R⁵, R⁷, R⁹ für Wasserstoff, geradkettiges oder verzweigtes C₁₋₈-Alkyl, das gegebenenfalls durch Hydroxy, C₁₋₄-Alkoxy, Phenoxy, Carboxy, 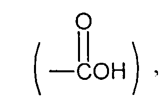 Carboxamid, 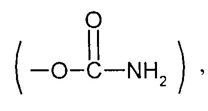 Imidazolyl, Indolyl, Guanidino, -SH
oder C₁₋₄-Alkylthio substituiert sein kann, ferner für gegebenenfalls substituiertes Phenyl, Benzyl oder Phenylethyl stehen,
R⁴, R⁶, R⁸, R¹⁰ für Wasserstoff, geradkettiges oder verzweigtes C₁₋₈-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für gegebenenfalls substituiertes Phenyl, Benzyl oder Phenylethyl stehen,
dadurch gekennzeichnet, daß man diese cyclischen Depsipeptide mit Sulfonylierungs-, Sulfenylierungs-, Thiocyanierungs- oder Phosphorylierungsreagenzien gegebenenfalls in Gegenwart von Katalysatoren, Hilfsstoffen und/oder Verdünnungsmitteln umsetzt.

4. Verfahren gemäß Anspruch 3, dadurch gekennzeichnet, daß als Depsipeptide Verbindungen der Formel (I) eingesetzt werden, in welcher R³ und R⁷ für Benzyl stehen und die übrigen Reste die in Anspruch 1 angegebene Bedeutung haben.

5. Verfahren gemäß Ansprüchen 3 und 4, dadurch gekennzeichnet, daß als Sulfonylierungs- und Sulfenylierungsreagenzien Halogensulfonsäuren (HalSO₃H), sowie deren Derivate, Dihalogensulfid, sowie seine Derivate, Halogensulfensäure, Sulfenylhalogenide, Disulfide, Schwefelsäure (Oleum) gegebenenfalls in einem gegen die Reagenzien inerten Verdünnungsmittel sowie gegebenenfalls in Gegenwart von Lewis-Säuren eingesetzt werden, und daß als Thiocyanierungsreagenzien Rhodanidsalze oder Dirhodan ((SCN)₂), gegebenenfalls in einem gegen die Reagenzien inerten Verdünnungsmittel sowie gegebenenfalls in Gegenwart von Lewis-Säuren eingesetzt werden, und daß als Phosphorylierungsreagenzien Phosphorhalogenide, gegebenenfalls in einem gegen die Reagenzien inerten Verdünnungsmittel sowie gegebenenfalls in Gegenwart von Lewis-Säuren eingesetzt werden.

6. Endoparasitizide Mittel, gekennzeichnet durch einen Gehalt an mindestens einem aromatischen sulfonylierten, sulfenylierten, thiocyanierten und/oder phosphorylierten cyclischen Depsipeptid gemäß Ansprüchen 1 bis 2.

7. Verwendung von aromatischen sulfonylierten, sulfenylierten, thiocyanierten und/oder phosphorylierten cyclischen Depsipeptiden gemäß Ansprüchen 1 bis 2 zur Herstellung von endoparasitiziden Mitteln.

## Claims

1. Compounds of the general formula (I) 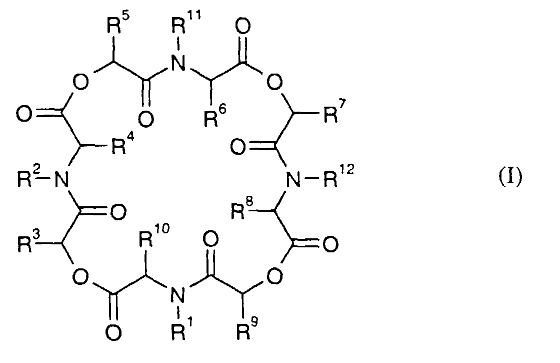 in which at least one of the radicals
R³, R⁴, R⁵, R⁶, R⁷, R⁸, R⁹ or R¹⁰ represents benzyl, which is substituted by a sulphonyl, sulphenyl, thiocyanato or phosphoryl radical
and in which otherwise the radicals
R¹, R², R¹¹ and R¹² represent hydrogen or optionally substituted C₁₋₈-alkyl, C₃₋₆-cycloalkyl, benzyl, phenylethyl or phenyl radicals,
R³, R⁵, R⁷ and R⁹ represent hydrogen, straight-chain or branched C₁₋₈-alkyl which is optionally substituted by hydroxyl, C₁₋₄-alkoxy, phenoxy, carboxyl, 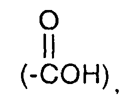 carboxamide, 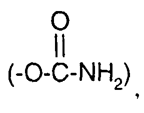 imidazolyl, indolyl, guanidino, -SH or C₁₋₄-alkylthio, and further represent optionally substituted phenyl, benzyl or phenylethyl,
R⁴, R⁶, R⁸ and R¹⁰ represent hydrogen, straight-chain or branched C₁₋₈-alkyl, C₂₋₆-alkenyl or C₃₋₇-cycloalkyl, each of which is optionally substituted by hydroxyl, C₁₋₄-alkoxy, carboxyl, carboxamide, imidazolyl, indolyl, guanidino, SH or C₁₋₄-alkylthio, or represent optionally substituted phenyl, benzyl or phenylethyl.

2. Compounds of the formula (I) according to Claim 1 in which the radicals R³ and R⁷ represent benzyl which is substituted by a sulphonyl, sulphenyl, thiocyanato or phosphoryl radical of the formulae -SO₂-A; -SO-A, -S-A, 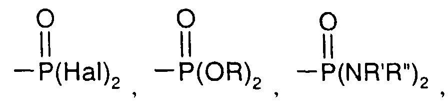 SCN wherein
Hal represents halogen,
A represents halogen, hydroxyl, -OR, -NH₂, -NHR', -NR'R",
R represents optionally substituted C₁₋₈-alkyl, C₂₋₆-alkenyl, phenyl, benzyl or phenylethyl,
R' represents optionally substituted C₁₋₈-alkyl, phenyl, benzyl or phenylethyl,
R" represents optionally substituted C₁₋₈-alkyl, phenyl, benzyl or phenylethyl,
and the radicals R' and R" represent together with the nitrogen atom attached a morpholine radical, piperazine radical, or N-methylpiperazine radical, and in which otherwise the radicals R¹, R², R⁴, R⁵, R⁶, R⁸, R⁹, R¹⁰, R¹¹, R¹² have the meanings stated in Claim 1;
the optionally substituted radicals may carry 1 to 2 identical or different substituents. Substituents which may be mentioned are:
alkyl having 1 to 4 carbon atoms, alkoxy having 1 to 4 carbon atoms, alkylthio having 1 to 4 carbon atoms, halogenoalkyl having 1 to 4 carbon atoms and 1 to 5 halogen atoms, where the halogen atoms are identical or different and represent fluorine, chlorine or bromine; hydroxyl; halogen, cyano; nitro; amino; monoalkyl- and dialkylamino having 1 to 4 carbon atoms per alkyl group.

3. Process for the aromatic sulphonylation, sulphenylation, thiocyanation and phosphorylation of cyclic depsipeptides of the general formula (I) 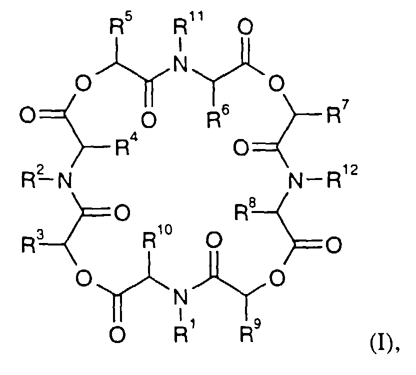 in which at least one of the radicals
R³, R⁴, R⁵, R⁶, R⁷, R⁸, R⁹, R¹⁰ represents benzyl,
and in which otherwise the radicals
R¹, R², R¹¹ and R¹² represent hydrogen, optionally substituted radicals C₁₋₈-alkyl, C₃₋₆-cycloalkyl, benzyl, phenylethyl or phenyl,
R³, R⁵, R⁷, R⁹ represent hydrogen, straight-chain or branched C₁₋₈-alkyl, which may optionally be substituted by hydroxyl, C₁₋₄-alkoxy, phenoxy, carboxyl, 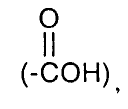 carboxamide, 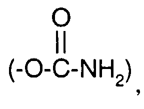 imidazolyl, indolyl, guanidino, -SH
or C₁₋₄-alkylthio, and furthermore represent optionally substituted phenyl, benzyl or phenylethyl,
R⁴, R⁶, R⁸, R¹⁰ represent hydrogen, straight-chain or branched C₁₋₈-alkyl, C₂₋₆-alkenyl, C₃₋₇-cycloalkyl, which may optionally be substituted by hydroxyl, C₁₋₄-alkoxy, carboxyl, carboxamide, imidazolyl, indolyl, guanidino, SH or C₁₋₄-alkylthio, and also represents optionally substituted phenyl, benzyl or phenylethyl,
characterized in that these cyclic depsipeptides are reacted with sulphonylating, sulphenylating, thiocyanating or phosphorylating agents, if appropriate in the presence of catalysts, auxiliaries and/or diluents.

4. Process according to Claim 3, characterized in that the depsipeptides used are compounds of the formula (I) in which R³ and R⁷ represent benzyl and the other radicals have the meanings stated in Claim 1.

5. Process according to Claims 3 and 4, characterized in that the sulphonylating and sulphenylating agents used are halosulphonic acids (HalSO₃H), and their derivatives, dihalogenosulphide, and its derivatives, halogenosulphenic acid, sulphenyl halides, disulphides, sulphuric acid (oleum) optionally in a diluent which is inert to the reagents and optionally in the presence of Lewis acids, and that the thiocyanating agents used are thiocyanate salts or thiocyanogen ((SCN)₂), optionally in a diluent which is inert to the reagents and optionally in the presence of Lewis acids, and that the phosphorylating agents used are phosphorus halides, optionally in a diluent which is inert to the reagents and optionally in the presence of Lewis acids.

6. Endoparasiticidal compositions, characterized in that they contain at least one aromatic sulphonylated, sulphenylated, thiocyanated and/or phosphorylated cyclic depsipeptide according to Claims 1 to 2.

7. Use of aromatic sulphonylated, sulphenylated, thiocyanated and/or phosphorylated cyclic depsipeptides according to Claims 1 to 2 for preparing endoparasiticidal compositions.

## Revendications

1. Composés répondant à la formule générale (I) 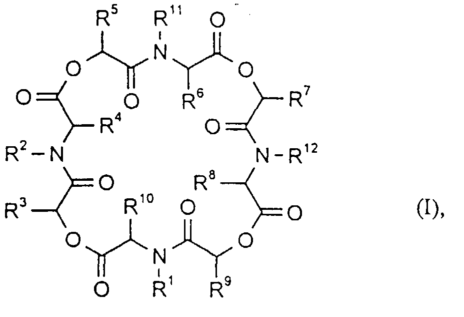 dans laquelle au moins un des radicaux
R³, R⁴, R⁵, R⁶, R⁷, R⁸, R⁹, R¹⁰ représente un groupe benzyle qui porte un ou plusieurs substituants identiques ou différents sulfonyle, sulfényle, thiocyano ou phosphoryle,
et dans laquelle par ailleurs les radicaux
R¹, R², R¹¹ et R¹² représentent un atome d'hydrogène, des radicaux alkyle en C₁-C₈, cycloalkyle en C₃-C₆, benzyle, phényléthyle, phényle le cas échéant substitués,
R³, R⁵, R⁷, R⁹ représentent un atome d'hydrogène, un groupe alkyle en C₁-C₈ à chaîne droite ou ramifiée qui peut porter le cas échéant un ou plusieurs substituants identiques ou différents hydroxyle, alcoxy en C₁-C₄, phénoxy, carboxyle, 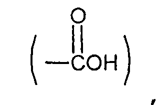 carboxamide, 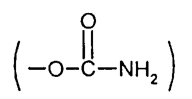 imidazolyle, indolyle, guanidino, -SH
ou alkyl(en C₁-C₄)thio, représentent en outre un groupe phényle, un groupe benzyle ou un groupe phényléthyle le cas échéant substitué,
R⁴, R⁶, R⁸, R¹⁰ représentent un atome d'hydrogène, un groupe alkyle en C₁-C₈ à chaîne droite ou ramifiée, un groupe alcényle en C₂-C₆, un groupe cycloalkyle en C₃-C₇ qui peuvent porter le cas échéant un ou plusieurs substituants identiques ou différents hydroxyle, alcoxy en C₁-C₄, carboxyle, carboxamide, imidazolyle, indolyle, guanidino, SH ou alkyl(en C₁-C₄)thio, et représentent un groupe phényle, un groupe benzyle ou un groupe phényléthyle le cas échéant substitué.

2. Composés répondant à la formule (I) selon la revendication 1, dans lesquels les radicaux R³ et R⁷ représentent un groupe benzyle qui peut porter un ou plusieurs substituants identiques ou différents sulfonyle, sulfényle, thiocyano ou phosphoryle répondant aux formules
-SO₂-A; SO-A, -S-A, 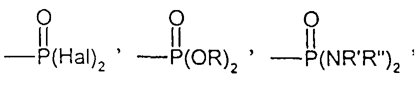 SCN dans lesquelles
Hal représente un atome d'halogène,
A représente un atome d'halogène, un groupe hydroxyle, un groupe -OR, un groupe -NH₂, un groupe -NHR', un groupe -NR'R",
R représente un groupe alkyle en C₁-C₈, un groupe alcényle en C₂-C₆, un groupe phényle, un groupe benzyle ou un groupe phényléthyle le cas échéant substitué,
R' représente un groupe alkyle en C₁-C₈, un groupe phényle, un groupe benzyle ou un groupe phényléthyle le cas échéant substitué,
R" représente un groupe alkyle en C₁-C₈, un groupe phényle, un groupe benzyle ou un groupe phényléthyle le cas échéant substitué,
et les radicaux R' et R" représentent, ensemble avec l'atome d'azote limitrophe, un radical morpholine, un radical pipérazine ou un radical N-méthylpipérazine, et dans laquelle par ailleurs les radicaux R¹, R², R⁴, R⁵, R⁶, R⁸, R⁹, R¹⁰, R¹¹, R¹² ont la signification indiquée à la revendication 1;
les radicaux le cas échéant substitués peuvent porter de 1 à 2 substituants identiques ou différents. A titre de substituants, on mentionnera:
un groupe alkyle contenant de 1 à 4 atomes de carbone, un groupe alcoxy contenant de 1 à 4 atomes de carbone, un groupe alkylthio contenant de 1 à 4 atomes de carbone, un groupe halogénalkyle contenant de 1 à 4 atomes de carbone et de 1 à 5 atomes d'halogène, les atomes d'halogène étant identiques ou différents et, à titre d'atomes d'halogène, on mentionnera un atome de fluor, un atome de chlore ou un atome de brome; un groupe hydroxyle; un atome d'halogène; un groupe cyano; un groupe nitro; un groupe amino; un groupe monoalkylamino et un groupe dialkylamino, chaque groupe alkyle portant de 1 à 4 atomes de carbone.

3. Procédé pour la sulfonylation, la sulfénylation, la thiocyanuration et la phosphorylation de depsipeptides cycliques répondant à la formule générale (I) 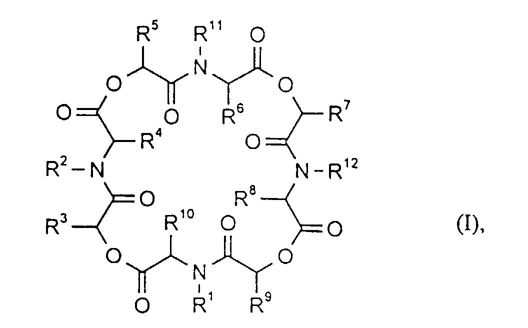 dans laquelle au moins un des radicaux
R³, R⁴, R⁵, R⁶, R⁷, R⁸, R⁹, R¹⁰ représente un groupe benzyle,
et dans laquelle par ailleurs les radicaux
R¹, R², R¹¹ et R¹² représentent un atome d'hydrogène, des radicaux alkyle en C₁-C₈, cycloalkyle en C₃-C₆, benzyle, phényléthyle ou phényle le cas échéant substitués,
R³, R⁵, R⁷, R⁹ représentent un atome d'hydrogène, un groupe alkyle en C₁-C₈ à chaîne droite ou ramifiée qui peut porter le cas échéant un ou plusieurs substituants identiques ou différents hydroxyle, alcoxy en C₁-C₄, phénoxy, carboxyle, 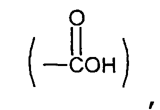 carboxamide, 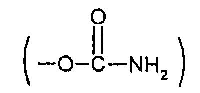 imidazolyle, indolyle, guanidino, -SH
ou alkyl(en C₁-C₄)thio, représentent en outre un groupe phényle, un groupe benzyle ou un groupe phényléthyle le cas échéant substitué,
R⁴, R⁶, R⁸, R¹⁰ représentent un atome d'hydrogène, un groupe alkyle en C₁-C₈ à chaîne droite ou ramifiée, un groupe alcényle en C₂-C₆, un groupe cycloalkyle en C₃-C₇ qui peuvent porter le cas échéant un ou plusieurs substituants identiques ou différents hydroxyle, alcoxy en C₁-C₄, carboxyle, carboxamide, imidazolyle, indolyle, guanidino, SH ou alkyl(en C₁-C₄)thio, et représentent un groupe phényle, un groupe benzyle ou un groupe phényléthyle le cas échéant substitué,
caractérisé en ce qu'on fait réagir ces depsipeptides cycliques avec des réactifs de sulfonylation, de sulfénylation, de thiocyanuration ou de phosphorylation le cas échéant en présence de catalyseurs, d'adjuvants et/ou de diluants.

4. Procédé selon la revendication 3, caractérisé en ce qu'on met en oeuvre, à titre de depsipeptides, des composés répondant à la formule (I) dans laquelle R³ et R⁷ représentent un groupe benzyle et les autres radicaux ont la signification indiquée à la revendication 1.

5. Procédé selon les revendications 3 et 4, caractérisé en ce qu'on met en oeuvre, à titre de réactifs de sulfonylation et de sulfénylation, des acides halogénosulfoniques (HalSO₃H), ainsi que leurs dérivés, des dihalogénosulfures, ainsi que leurs dérivés, des acides halogénosulféniques, des halogénures de sulfényle, des disulfures, de l'acide sulfurique (Oleum), le cas échéant dans un diluant inerte vis-à-vis des réactifs et le cas échéant en présence d'acides de Lewis, et en ce qu'on met en oeuvre, à titre de réactifs de thiocyanuration, des sels de sulfocyanure ou le disulfocyanure ((SCN)₂), le cas échéant dans un solvant inerte vis-à-vis des réactifs, ainsi que le cas échéant en présence d'acides de Lewis, et en ce qu'on met en oeuvre, à titre de réactifs de phosphorylation, des halogénures de phosphore le cas échéant dans un diluant inerte vis-à-vis des réactifs et le cas échéant en présence d'acides de Lewis.

6. Agents endoparasiticides caractérisés par une teneur en au moins un depsipeptide cyclique aromatique sulfonylé, sulfénylé, thiocyanuré et/ou phosphorylé selon les revendications 1 à 2.

7. Utilisation de depsipeptides cycliques aromatiques sulfonylés, sulfénylés, thiocyanurés et/ou phosphorylés selon les revendications 1 à 2 pour la préparation d'agents endoparasiticides.
